# EUROPEAN PATENT APPLICATION

(11) **EP 3 510 934 A1**
(43) Date of publication of application: **17.07.2019**
(21) Application number: 18176404.4
(22) Date of filing: 07.06.2018
(51) Int. Cl.: A61B 8/00, B06B 1/06

(54) **ULTRASONIC PROBE**

(30) Priority: 10.01.2018 KR 20180003165
(71) Applicant: Samsung Medison Co., Ltd., Hongcheon-gun, Gangwon-do, 25108 (KR)
(72) Inventor: JUNG, Jin Woo, Seoul (KR)
(74) Representative: Grootscholten, Johannes A.M.

(57) **Abstract**

Disclosed herein is a multi-row ultrasonic probe having improved heat dissipation capability. An ultrasonic probe includes a transducer configured to generate ultrasound and having multiple rows, a kerf dividing the transducer into multiple rows, and a heat dissipation member located inside the kerf and transferring heat generated by the transducer to the outside.

## Description

### CROSS-REFERENCE TO RELATED APPLICATION(S)

This application claims the benefit of Korean Patent Application No. 10-2018-0003165, filed on January 10, 2018 in the Korean Intellectual Property Office, the disclosure of which is incorporated herein by reference.

### BACKGROUND

### 1. Field

Embodiments of the present disclosure relate to an ultrasonic probe to generate an image of the inside of an object by using ultrasound, and more particularly, to a multi-row ultrasonic probe having improved heat dissipation capability.

### 2. Description of the Related Art

An ultrasound imaging apparatus radiates ultrasonic signals toward a target region inside an object from a surface of the object and then collects reflected ultrasonic signals (ultrasonic echo signals) to non-invasively acquire tomograms of soft tissues or images of blood streams using information thereon.

Ultrasound imaging apparatuses are relatively small in size and inexpensive, display an image in real time, and have high safety due to no radiation exposure as compared with other diagnostic imaging apparatuses such as X-ray diagnosis apparatuses, computerized tomography (CT) scanners, magnetic resonance imaging (MRI) apparatuses, and nuclear medicine diagnosis apparatuses. Thus, ultrasound imaging apparatuses have been widely used for heart diagnosis, celiac diagnosis, urinary diagnosis, and obstetric diagnosis.

An ultrasound imaging apparatus includes an ultrasonic probe that transmits ultrasonic signals to an object and receives ultrasonic echo signals reflected by the object to acquire an ultrasound image of the object and a main body that generates an image of the inside of the object by using the ultrasonic echo signals received by the ultrasonic probe.

A conventional single-row (1D) probe has a fixed focus due to a curvature of a lens, and thus a focal range thereof is limited.

To remove the limitation in the focal range, a multi-row (1.25D to 1.75D) probe has been developed. Since a focus area of the multi-row probe is physically or electrically adjusted, a high resolution image may be realized in a wider area. Thus, recently, a multi-row probe of 1.25D (with three rows) or higher has replaced the single-row (1D) probe.

Meanwhile, only a part of electrical energy applied to drive the ultrasonic probe is converted into acoustic energy, and a considerable amount of the electrical energy is converted into heat.

When the ultrasonic probe is used for medical purposes, the ultrasonic probe is in direct contact with a human body, and thus a surface temperature of the ultrasonic probe is limited to a predetermined temperature or less for safety reasons.

Therefore, there is a need to develop a heat dissipation structure to reduce the surface temperature of the ultrasonic probe.

### SUMMARY

Therefore, it is an aspect of the present disclosure to provide a multi-row ultrasonic probe having improved heat dissipation capability.

It is another aspect of the present disclosure to provide a multi-row ultrasonic probe having improved safety by reducing a surface temperature thereof.

Additional aspects of the disclosure will be set forth in part in the description which follows and, in part, will be obvious from the description, or may be learned by practice of the disclosure.

In accordance with one aspect of the present disclosure, an ultrasonic probe includes a transducer configured to generate ultrasound and having multiple rows, a kerf dividing the transducer into multiple rows, and a heat dissipation member located inside the kerf and transferring heat generated by the transducer to the outside.

A width of the heat dissipation member may be equal to or smaller than that of the kerf.

The heat dissipation member may have a thermal conductivity of 0.5 W/m.K or greater.

The heat dissipation member may include a coating layer covering an outer surface of the heat dissipation member and formed of a material comprising at least one of a non-metallic material, silicone, an epoxy resin, and parylene to reduce electrical conductivity of the heat dissipation member.

The ultrasonic probe may further include a heat dissipation housing provided to cover an outer periphery of the transducer, wherein the heat dissipation member may be connected to the heat dissipation housing to transfer heat of the heat dissipation member to the heat dissipation housing.

The ultrasonic probe may further include a matching layer provided on an upper surface of the transducer.

The kerf may include a first kerf dividing the transducer into rows, and a second kerf dividing the matching layer in the same direction as a direction in which the first kerf divides the transducer.

A height of the heat dissipation member may be greater than a height of the transducer and equal to or smaller than a sum of heights of the transducer and the matching layer.

The ultrasonic probe may further include a backing layer provided on a lower surface of the transducer.

The kerf may further include a third kerf dividing the backing layer in the same direction as a direction in which the first kerf divides the transducer.

The height of the heat dissipation member may be greater than the height of the transducer and equal to or smaller than a sum of heights of the transducer, the matching layer, and the backing layer.

The kerf may be provided plural in number, and the heat dissipation member is located inside each of the plurality of kerfs.

A plurality of heat dissipation members may be located inside each of the plurality of kerfs.

A height of the heat dissipation member may be greater than a width of the heat dissipation member.

The heat dissipation member may include a plurality of heat dissipation wires arranged in parallel in directions direction crossing the elevation direction and the azimuth direction, respectively.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a diagram illustrating a part of an ultrasonic probe according to an embodiment.
FIG. 2 is a diagram illustrating another part of the ultrasonic probe in an angle different from that of FIG. 1.
FIG. 3 is a diagram illustrating only a heat dissipation member and a heat dissipation frame of an ultrasonic probe according to an embodiment.
FIG. 4 is a cross-sectional view illustrating a structure of an ultrasonic probe according to an embodiment.
FIG. 5 is a cross-sectional view illustrating a structure of an ultrasonic probe according to another embodiment.
FIG. 6 is a cross-sectional view illustrating a structure of an ultrasonic probe according to another embodiment.
FIG. 7 is a cross-sectional view illustrating a structure of an ultrasonic probe according to another embodiment.
FIG. 8 is a cross-sectional view illustrating a structure of an ultrasonic probe according to another embodiment.
FIG. 9 is a cross-sectional view illustrating a structure of an ultrasonic probe according to another embodiment.

### DETAILED DESCRIPTION

Reference will now be made in detail to the embodiments of the present disclosure, examples of which are illustrated in the accompanying drawings, wherein like reference numerals refer to like elements throughout.

The terms used in the present specification are merely used to describe particular embodiments, and are not intended to limit the present disclosure. An expression used in the singular encompasses the expression of the plural, unless it has a clearly different meaning in the context. In the present specification, it is to be understood that the terms such as "including" or "having," etc., are intended to indicate the existence of the features, numbers, operations, components, parts, or combinations thereof disclosed in the specification, and are not intended to preclude the possibility that one or more other features, numbers, operations, components, parts, or combinations thereof may exist or may be added.

It will be understood that, although the terms "first", "second", etc., may be used herein to describe various elements, these elements should not be limited by these terms. The above terms are used only to distinguish one component from another. For example, a first component discussed below could be termed a second component, and similarly, the second component may be termed the first component without departing from the teachings of this disclosure. As used herein, the term "and/or" includes any and all combinations of one or more of the associated listed items.

An ultrasonic probe according to an embodiment of the present disclosure may be implemented using a multi-row ultrasonic probe.

A single-row (1D) probe includes a transducer divided into a plurality of elements in either an azimuth direction or an elevation direction.

For example, in a single-row (1D) probe including a transducer divided into a plurality of elements in the azimuth direction, the transducer is not divided in the elevation direction.

Since such single-row probes have a physical focus area fixed by a curvature of a lens, the focus area is limited.

Research has been conducted on multi-row probes to remove the limitation of the focus area.

A multi-row probe includes a transducer divided in both the azimuth direction and the elevation direction.

For example, a 1.25D probe including a transducer divided into a plurality of elements in the azimuth direction is divided into three rows in the elevation direction. A 1.5D probe including a transducer divided into a plurality of elements in the same direction is divided into four rows in the elevation direction. A 1.75D probe is divided into five rows in the elevation direction.

A 2D probe includes a transducer divided into a plurality of elements in both the azimuth direction and the elevation direction.

Such a multi-row probe may realize high resolution images in a wider area since a focus area may be physically and electrically adjusted.

An ultrasonic probe is an apparatus that generates ultrasound by converting electrical energy into acoustic energy. In a process of converting electrical energy into acoustic energy, all of the electrical energy is not converted into acoustic energy and a large amount of electrical energy is converted into thermal energy. The thermal energy may increase temperature of a surface of the ultrasonic probe.

When the ultrasonic probe is used for medical purposes, a surface of the ultrasonic probe is in direct contact with a human body, and thus safety issues such as burns may occur. To prevent such issues in advance, there is limitations to the surface temperature of the ultrasonic probe. Thus, there is a need to develop an efficient heat dissipation structure to reduce the surface temperature of the ultrasonic probe.

Hereinafter, an ultrasonic probe according to an embodiment will be described in detail with reference to the accompanying drawings.

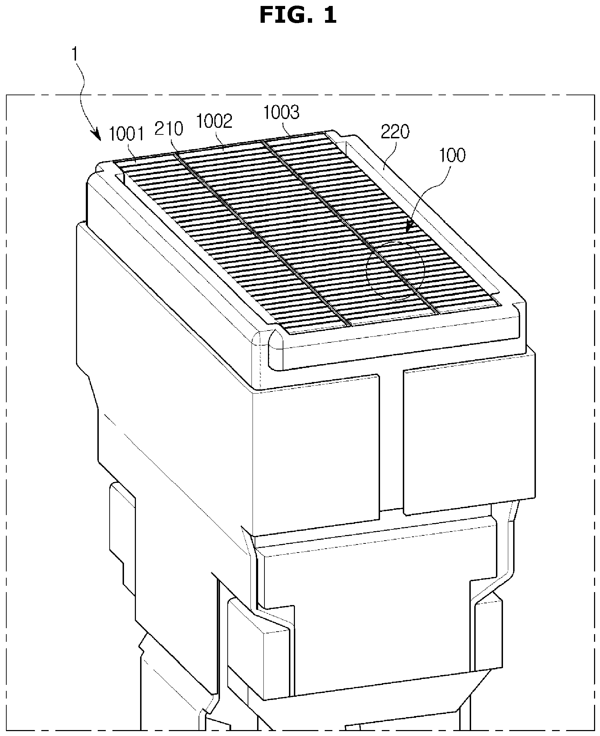

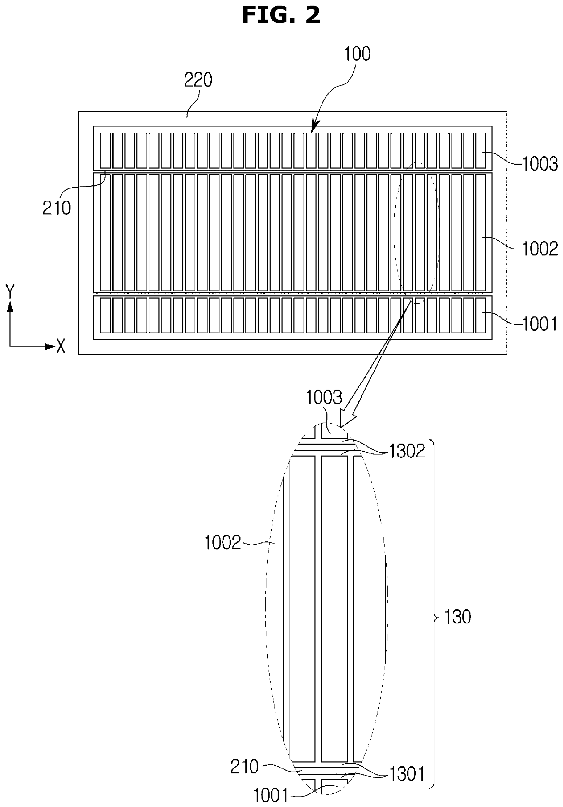

FIG. 1 is a diagram illustrating a part of an ultrasonic probe according to an embodiment. FIG. 2 is a diagram illustrating another part of the ultrasonic probe in an angle different from that of FIG. 1.

Meanwhile, although a 1.25D probe will be described hereinafter as an example of a multi-row probe, the embodiment is not limited thereto. The multi-row probe may also include a 1.5D probe, a 1.75D probe, and a 2D probe as well as the 1.25D probe.

As illustrated in FIGS. 1 and 2, an ultrasonic probe 1 according to an embodiment may include a transducer 100 divided into a plurality of elements in the elevation direction (X-direction in FIG. 2). In addition, the transducer 100 of the ultrasonic probe 1 may be divided into three rows in the azimuth direction (Y-direction in FIG. 2). Although not shown in the drawings, a transducer of the 1.5D probe may be divided into four rows and a transducer of the 1.75D probe may be divided into five rows.

The transducer 100 may have a kerf 130. The kerf 130 may divide the transducer 100 such that the transducer 100 has multiple rows. In other words, the kerf 130 may refer to a gap formed between rows of the transducer 100.

As illustrated in FIGS. 1 and 2, a transducer 100 of the 1.25D probe may have two kerfs 1301 and 1302. Although not shown in the drawings, the 1.5D probe may have three kerfs, and the 1.75D probe may have four kerfs.

According to an embodiment, a heat dissipation member 210 may be located in the kerf 130 defining multiple rows. As illustrated in the drawings, the heat dissipation member 210 may be disposed in each of the plurality of kerfs 1301 and 1302. Alternatively, the heat dissipation member 210 may be disposed in only one of the plurality of kerfs 1301 and 1302.

The heat dissipation member 210 may be located to be adjacent to the transducer 100 serving as a heat source and absorb heat. Particularly, the heat dissipation member 210 may be arranged between two adjacent rows (e.g., rows 1001 and 1002) of the transducer 100 and absorb heat. Thus, the heat dissipation member 210 may receive heat directly from the transducer 100 serving as a heat source. In other words, the heat dissipation member 210 may absorb heat directly from the transducer 100.

The heat dissipation member 210 may be formed of a material having a high thermal conductivity. For example, the heat dissipation member 210 may be formed of a composite material including a metal such as copper (Cu) or metal powder.

The heat dissipation member 210 may be located between two adjacent rows (e.g., rows 1001 and 1002). The heat dissipation member 210 may be located in the kerf 130 separating two adjacent rows from each other.

The heat dissipation member 210 be in contact with the two rows located at both sides of the heat dissipation member 210. In this regard, when the heat dissipation member 210 has electrical conductivity, an electrical short may occur between two adjacent rows. In this case, the probe cannot function as a multi-row probe, and thus there is a need to prevent electrical shorts between adjacent rows. To prevent the electrical shorts, the heat dissipation member 210 may include a thin film coating layer (not shown) such as a Parylene coating not to have electrical conductivity.

As illustrated in FIGS. 1 and 2, the ultrasonic probe 1 may include a heat dissipation housing 220 connected to the heat dissipation member 210. For example, both ends of the heat dissipation member 210 may be connected to the heat dissipation housing 220. The heat dissipation member 210 may be connected the heat dissipation housing 220 to transfer heat from the heat dissipation member 210 to the heat dissipation housing 220. Although the both ends of the heat dissipation member 210 are connected to the heat dissipation housing 220 in FIGS. 1 and 2, the embodiment is not limited thereto. The heat dissipation member 210 may be connected to the heat dissipation housing 220 at any portion. The heat dissipation housing 220 may be provided to surround side surfaces of the transducer 100. The heat dissipation housing 220 may absorb heat generated in the side surfaces of the transducer 100 and release the heat to the outside. In addition, the heat dissipation housing 220 may receive heat from the heat dissipation member 210 and release the heat to the outside.

As described above, since the transducer 100 is arranged at both sides of the heat dissipation member 210, heat sources are located on both side surfaces thereof. On the other hand, the transducer 100 is arranged on inner surfaces of the heat dissipation housing 220 is and a housing (not shown) or external air is located on outer surfaces thereof. Thus, a temperature of the heat dissipation member 210 may be increased by heat generated by the transducer 100 to be higher than that of the heat dissipation housing 220. The heat dissipation housing 220 may release heat received from the heat dissipation member 210 to the housing (not shown) or external air.

The heat dissipation member 210 may be formed of a material having a high heat transfer efficiency. For example, the heat dissipation member 210 may be formed of a metallic material. The heat dissipation member 210 may be formed of a material having a thermal conductivity of 0.5 W/m.K or higher. The heat dissipation member 210 may be formed of a single metal or a composite metal to obtain a thermal conductivity of 0.5 W/m.K or higher. In addition, the heat dissipation member 210 may include at least one of metal particles, carbon nanotube, and graphene.

The heat dissipation housing 220 may be integrated with the heat dissipation member 210. The heat dissipation housing 220 may also be formed of a material having a high thermal conductivity like the heat dissipation member. For example, the heat dissipation housing 220 may be formed of a composite material including a metal such as copper (Cu) or metal powder. The heat dissipation housing 220 may be formed of a material having a thermal conductivity of 0.5 W/m.K or higher.

The heat dissipation housing 220 may be provided to cover side surfaces of the transducer 100. The heat dissipation housing 220 may be in contact with both outer rows 1001 and 1003 of the transducer 100. In this case, when the heat dissipation housing 220 has electric conductivity, an electrical short may occur between the both outer rows 1001 and 1003. In this case, the probe may not function as a multi-row probe, the heat dissipation housing 220 may not have electrical conductivity. To this end, the heat dissipation housing 220 may have a thin film coating layer (not shown) such as a Parylene coating on the surface thereof.

When the heat dissipation member 210 is formed of a metallic material or a material having electrical conductivity, the coating layer may be provided thereon to reduce the electrical conductivity of the heat dissipation member 210. Thus, the coating layer may be formed of a material having a low electrical conductivity. For example, the coating layer may be formed of a material including at least one of a non-metallic material, silicone, an epoxy resin, and parylene.

Various methods of reducing electrical conductivity of the heat dissipation member 210 may be used. In addition to the coating, a surface treating process such as painting and film formation may be performed. More particularly, the methods may include fluorocarbon resin coating and anodizing (anodic oxidation).

According to an embodiment, heat absorbed by the heat dissipation member 210 may be released to the outside via the heat dissipation housing 220. As described above, the heat dissipation member 210 absorbs heat directly from the transducer 100 that is a heat source and releases the heat through the heat dissipation housing 220. Since the heat dissipation member 210 and the heat dissipation housing 220 absorb heat directly from the transducer 100 and releases the heat to the outside. Thus, heat dissipation efficiency may be increased according to an embodiment in comparison with a method of indirectly absorbing heat and releasing the heat.

Meanwhile, although the heat dissipation member is disposed in the kerf 130 defining the multiple rows, there is almost no change in acoustic characteristics of the ultrasonic probe. Accordingly, the ultrasonic probe according to an embodiment may improve heat dissipation performance with no substantial changes in performance.

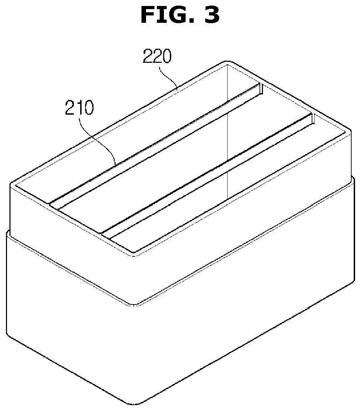

FIG. 3 is a diagram illustrating only a heat dissipation member and a heat dissipation frame of an ultrasonic probe according to an embodiment.

As illustrated in FIG. 3, the heat dissipation member 210 may be integrated with the heat dissipation housing 220. However, the embodiment is not limited thereto, and the heat dissipation member 210 and the heat dissipation housing 220 may be separated from each other and may be formed of different materials.

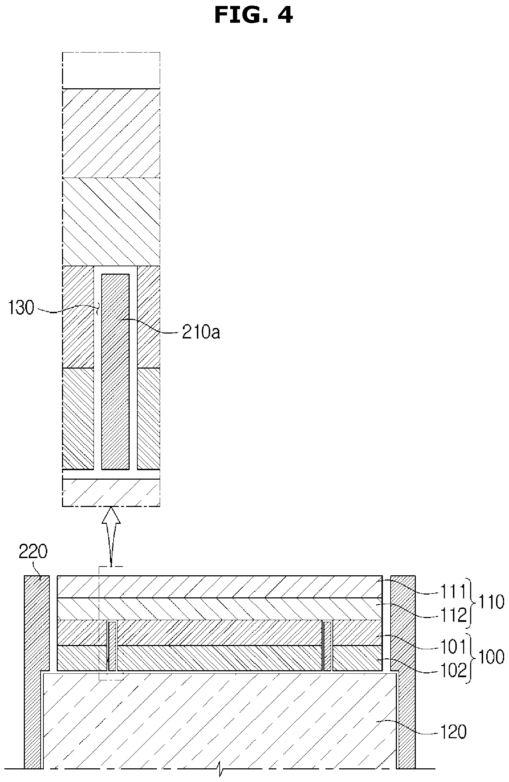

FIG. 4 is a cross-sectional view illustrating a structure of an ultrasonic probe according to an embodiment.

As illustrated in FIG. 4, the ultrasonic probe according to an embodiment may include a transducer 100, a matching layer 110 provided on an upper surface of the transducer 100, and a backing layer 120 provided on a lower surface of the transducer 100. In this regard, upper and lower directions are defined based on FIG. 4.

Examples of the transducer may include a magnetostrictive ultrasonic transducer that uses a magnetostrictive effect of a magnetic material, a capacitive micromachined ultrasonic transducer that transmits and receives ultrasound by using oscillation of hundreds or thousands of micromachined thin films, and a piezoelectric ultrasonic transducer that uses piezoelectric effects of a piezoelectric material. Hereinafter, a piezoelectric ultrasonic transducer will be described as an example of the transducer according to an embodiment.

A phenomenon in which a voltage is generated by a mechanical pressure applied to a material is referred to as piezoelectric effect and a phenomenon in which a material is mechanically deformed by a voltage applied thereto is referred to as inverse piezoelectric effect. A material exhibiting these effects is referred to as a piezoelectric material.

That is, a piezoelectric material is a material that coverts electrical energy into mechanical vibration energy and converts mechanical vibration energy into electrical energy.

The transducer 100 according to an embodiment may include a piezoelectric layer 101 including a piezoelectric material that generates ultrasound by converting an electrical signal applied thereto into a mechanical vibration and an acoustic layer 102 provided on a rear surface of the piezoelectric layer 101.

The piezoelectric material constituting the piezoelectric layer 101 may include a ceramic of lead zirconate titanate (PZT), a PZMT single crystal including a solid solution of lead magnesium niobate and lead titanate, or a PZNT single crystal including a solid solution of lead zinc niobate and lead titanate.

The acoustic layer 102 may have a higher acoustic impedance than the piezoelectric layer 101. The acoustic layer 102 may be formed of a material having electrical conductivity. In addition, a thickness of the acoustic layer 102 may be 1/2, 1/4, 1/8, or 1/16 of a wavelength of the piezoelectric material constituting the piezoelectric layer 101. That is, when a wavelength of the piezoelectric material constituting the piezoelectric layer 101 is λ, the thickness of the acoustic layer 102 may be 1/2 λ, 1/4 λ, 1/8 λ, or 1/16 λ.

According to an embodiment, the acoustic layer 102 may be an acoustic reflector plate. The acoustic reflector plate may be provided in front of the backing layer 120. The acoustic reflector plate may totally reflect ultrasound proceeding toward the backing layer 120. Thus, the ultrasonic probe may have increased bandwidth and improved sensitivity.

The acoustic layer 102 may be formed of a material having a very high acoustic impedance. For example, the acoustic layer 102 may be formed of at least one of tungsten carbide and a graphite composite material.

Electrodes to which electrical signals are applied may be provided on the upper and lower surfaces of the transducer 100. A ground electrode (not shown) may be provided on a front surface of the transducer 100, and a signal electrode (not shown) may be provided on a rear surface of the transducer 100. The ground electrode and the signal electrode may be provided as a flexible printed circuit board, respectively.

The matching layer 110 may be provided on the upper surface of the transducer 100. The matching layer 110 matches an acoustic impedance of the transducer 100 to that of the object by reducing difference of acoustic impedances between the transducer 100 and the object such that ultrasound generated by the transducer 100 is efficiently transmitted to the object.

To this end, the matching layer 110 may have a median between the acoustic impedance of the transducer 100 and that of the object. Particularly, the matching layer 110 may have a median between the acoustic impedance of the piezoelectric layer 101 and that of the object.

In addition, the matching layer 110 may include a plurality of matching layers such that the acoustic impedance is changed in a stepwise manner from the transducer 100 to the object. As illustrated in FIG. 4, the matching layer 110 may include a first matching layer 111 and a second matching layer 112. The plurality of matching layers may be formed of different materials. Meanwhile, the matching layer 110 may be formed of glass or resin.

Although not shown in the drawings, a lens may be installed at the upper surface of the matching layer 110. The lens may be arranged on the upper portion of the first matching layer 111 in direct contact with the object (not shown). The lens may focus ultrasound generated by the piezoelectric layer 101. The lens may include a material having an acoustic impedance similar to that of the object, such as silicone and rubber. The lens may be implemented using a convex type lens having an outwardly curved central portion or a linear type lens having a flat surface.

The backing layer 120 may be provided on the lower surface of the transducer 100. The backing layer 120 reduces a pulse width of ultrasound by inhibiting free vibration of the piezoelectric layer 101 and prevents ultrasound from proceeding backward from the piezoelectric layer 101. Thus, the backing layer 120 may prevent image distortion. The backing layer 120 may be formed of an epoxy resin and a material including rubber provided with tungsten powder.

Referring to FIG. 4, the heat dissipation member 210 according to an embodiment may be located in the transducer 100.

The kerf 130 may divide the transducer 100 into multiple rows.

As described above, the transducer 100 may include the piezoelectric layer 101 and the acoustic layer 102. The kerf 130 may divide each of the piezoelectric layer 101 and the acoustic layer 102.

The heat dissipation member 210 may be provided inside the kerf 130. A width of the heat dissipation member 210 may be equal to or smaller than that of the kerf 130. When the width of the heat dissipation member 210 is greater than that of the kerf 130, the heat dissipation member 210 cannot be located inside the kerf 130 without changing the width of the kerf 130. As described above, since the width of the heat dissipation member 210 is smaller than that of the kerf 130, the heat dissipation member 210 may be located inside the kerf 130. Meanwhile, the width refers to a length in the transverse direction in FIG. 4.

The cross-section of the heat dissipation member 210 may be formed in an approximately rectangular shape. However, the embodiment is not limited thereto, and the cross-section of the heat dissipation member 210 may be formed in various shapes such as a circular, oval, or square shape.

A height of the heat dissipation member 210 may be equal to or smaller than that of the transducer 100. As illustrated in FIG. 4, the matching layer 110 provided on the upper surface of the transducer 100 and the backing layer 120 provided on the lower surface of the transducer 100 may be divided in the elevation direction but not in the azimuth direction. Thus, when the height of the heat dissipation member 210 is greater than that of the transducer 100, the heat dissipation member 210 cannot be located between the matching layer 110 and the backing layer 120. As described above, since the height of the heat dissipation member 210 is equal to or smaller than that of the transducer 100, the heat dissipation member 210 may be located between the matching layer 110 and the backing layer 120. Meanwhile, the height refers to a length in the longitudinal direction in FIG. 4.

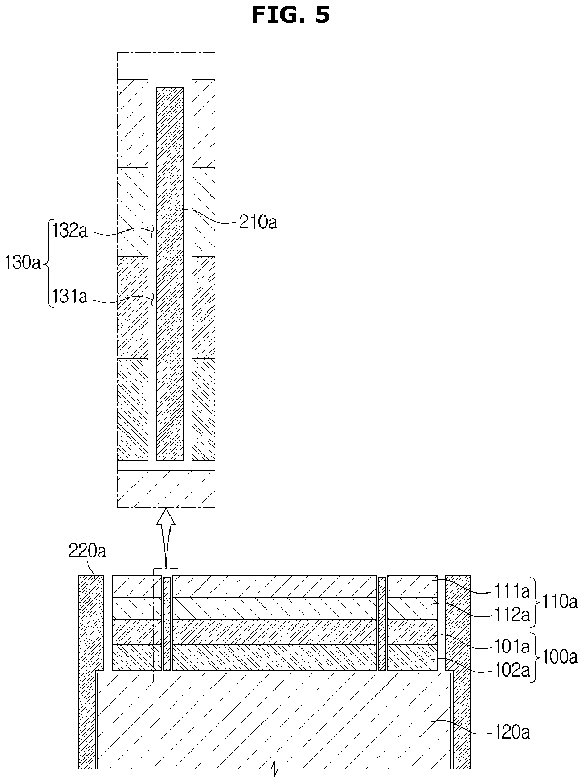

FIG. 5 is a cross-sectional view illustrating a structure of an ultrasonic probe according to another embodiment.

Hereinafter, descriptions given above with reference to FIGS. 1 to 4 will not be repeated.

Referring to FIG. 5, not only a transducer 100a but also a matching layer 110a may be divided in the azimuth direction.

A kerf 130a may include a first kerf 131a dividing the transducer 100a in the azimuth direction and a second kerf 132a dividing the matching layer 110a in the azimuth direction.

A heat dissipation member 210a may be located not only in the first kerf 131a but also in the second kerf 132a. A width of the heat dissipation member 210a may be smaller than those of the first kerf 131a and the second kerf 132a. A height of the heat dissipation member 210a may be greater than that of the transducer 100a. The height of the heat dissipation member 210a may be equal to or smaller than a sum of heights of the transducer 100a and the matching layer 110a. Thus, the heat dissipation member 210a may be located inside the first kerf 131a and the second kerf 132a. Meanwhile, the first kerf 131a and the second kerf 132a may have the same width.

As the height of the heat dissipation member 210a increases, a surface area of the heat dissipation member 210a increases. Thus, an amount of heat absorbed by the heat dissipation member 210a may increase. As the amount of heat absorbed by the heat dissipation member 210a increases, heat dissipation capability of the heat dissipation member 210a and a heat dissipation housing 220a may be improved, and therefore the surface of the ultrasonic probe may be cooled more efficiently.

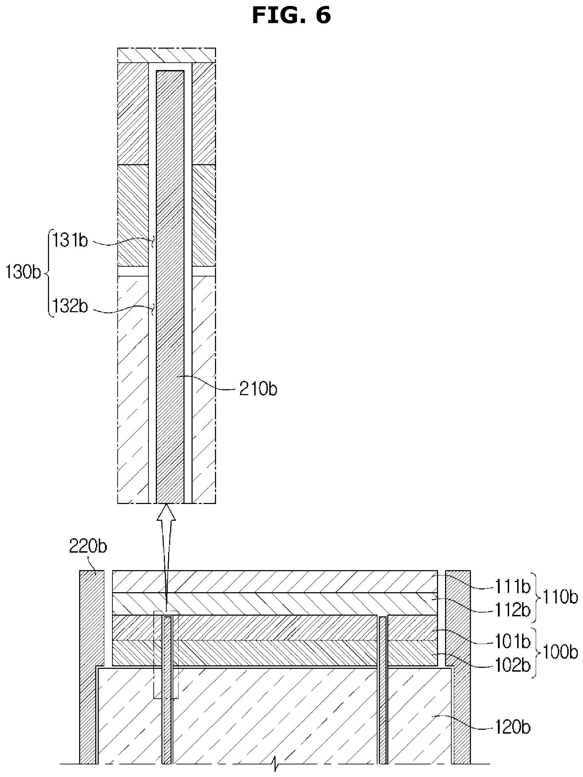

FIG. 6 is a cross-sectional view illustrating a structure of an ultrasonic probe according to another embodiment.

Referring to FIG. 6, not only a transducer 100b but also a backing layer 120b may be divided in the azimuth direction.

A kerf 130b may include a first kerf 131b dividing the transducer 100b in the azimuth direction and a second kerf 132b dividing the backing layer 120b in the azimuth direction.

A heat dissipation member 210b may be located not only in the first kerf 131b but also in the second kerf 132b. A width of the heat dissipation member 210b may be smaller than those of the first kerf 131b and the second kerf 132b. A height of the heat dissipation member 210b may be greater than that of the transducer 100b. The height of the heat dissipation member 210b may be equal to or smaller than a sum of heights of the transducer 100b and the backing layer 120b. Thus, the heat dissipation member 210b may be located inside the first kerf 131b and the second kerf 132b.

As the height of the heat dissipation member 210b increases, a surface area of the heat dissipation member 210b increases. Thus, an amount of heat absorbed by the heat dissipation member 210b may increase. As the amount of heat absorbed by the heat dissipation member 210b increases, heat dissipation capability of the heat dissipation member 210b and a heat dissipation housing 220b may be improved, and therefore the surface of the ultrasonic probe may be cooled more efficiently.

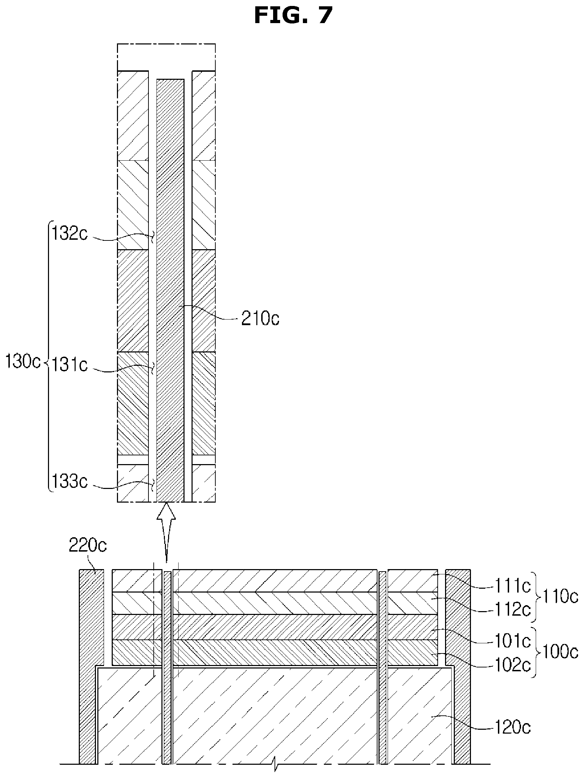

FIG. 7 is a cross-sectional view illustrating a structure of an ultrasonic probe according to another embodiment.

Referring to FIG. 7, not only a transducer 100c but also a matching layer 110c and a backing layer 120c may be divided in the azimuth direction.

A kerf 130c may include a first kerf 131c dividing the transducer 100c in the azimuth direction, a second kerf 132c dividing the matching layer 110c in the azimuth direction, and a third kerf 133c dividing the backing layer 120c in the azimuth direction.

A heat dissipation member 210c may be located in the first kerf 131c, the second kerf 132c, and the third kerf 133c. A width of the heat dissipation member 210c may be smaller than those of the first kerf 131c, the second kerf 132c, and the third kerf 133c. A height of the heat dissipation member 210c may be greater than that of the transducer 100c. The height of the heat dissipation member 210c may be equal to or smaller than a sum of heights of the transducer 100c, the matching layer 110c, and the backing layer 120c. Thus, the heat dissipation member 210c may be located inside the first kerf 131c, the second kerf 132c, and the third kerf 133c.

As the height of the heat dissipation member 210c increases, a surface area of the heat dissipation member 210c increases. Thus, an amount of heat absorbed by the heat dissipation member 210c may increase. As the amount of heat absorbed by the heat dissipation member 210c increases, heat dissipation capability of the heat dissipation member 210c and the heat dissipation housing 220c may be improved, and thus the surface of the ultrasonic probe may be cooled more efficiently.

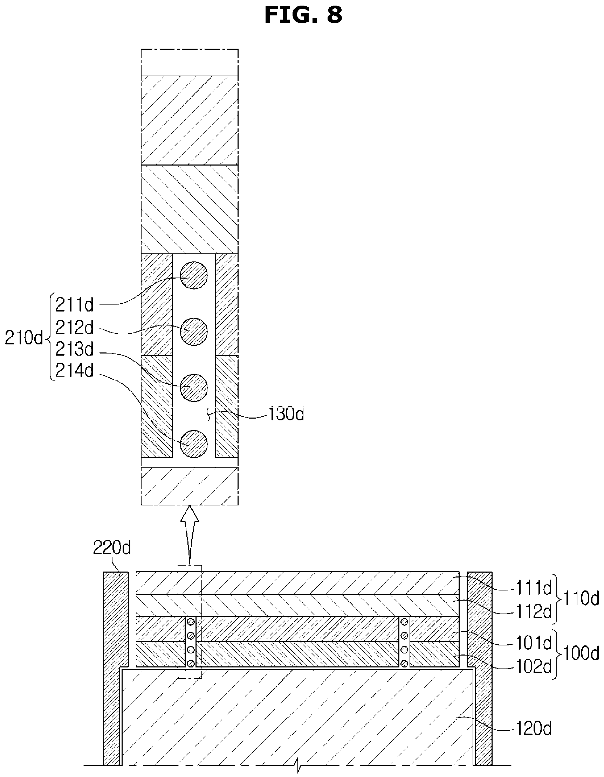

FIG. 8 is a cross-sectional view illustrating a structure of an ultrasonic probe according to another embodiment.

Referring to FIG. 8, a heat dissipation member 210d may be provided in the form of a plurality of wires. Characteristics of materials of the heat dissipation member 210d other than the shape and number are the same as those described above, and thus detailed descriptions thereof will not be repeated.

The heat dissipation member 210d may include a plurality of wires 211d, 212d, 213d, and 214d. Although FIG. 8 illustrates four wires, the number of wires may vary. The heat dissipation member may include two or three wires, alternatively more than five wires.

In addition, although FIG. 8 illustrates that only a transducer 100d is divided in the azimuth direction, a matching layer 110d and/or a backing layer 120d may also be divided in the azimuth direction. In this case, the plurality of wires may be arranged not only in the kerf dividing the transducer but also in the kerfs dividing the matching layer and/or the backing layer.

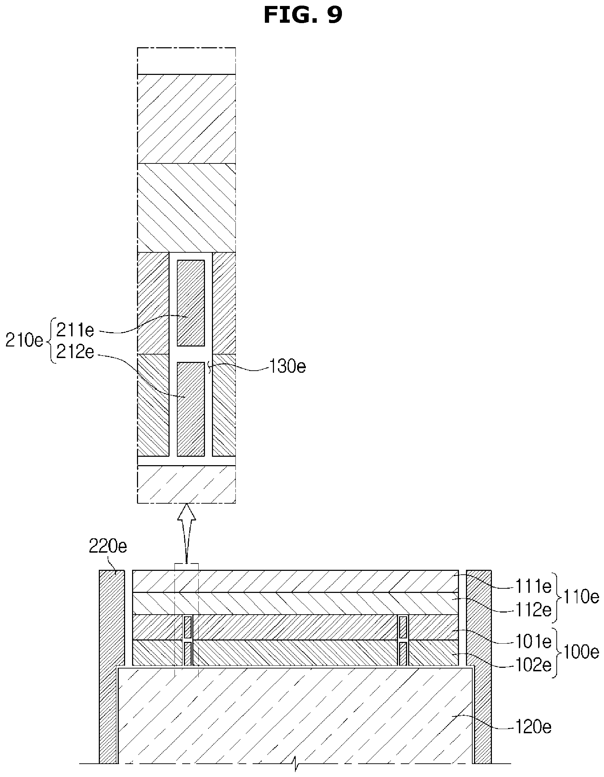

FIG. 9 is a cross-sectional view illustrating a structure of an ultrasonic probe according to another embodiment.

Referring to FIG. 9, a heat dissipation member 210e may be provided plural in number. Characteristics of materials of the heat dissipation member 210e other than the shape and number are the same as those described above, and thus detailed descriptions thereof will not be repeated.

The heat dissipation member 210e may include a plurality of heat dissipation members 211e and 212e. Although FIG. 9 illustrates two heat dissipation members provided in the plate form, the number may vary. Also, three or more heat dissipation members may be provided.

In addition, although FIG. 9 illustrates that only a transducer 100e is divided in the azimuth direction, a matching layer 110e and/or a backing layer 120e may also be divided in the azimuth direction. In this case, the plurality of plates may be arranged not only in the kerf dividing the transducer but also in the kerfs dividing the matching layer and/or the backing layer.

As is apparent from the above description, a multi-row ultrasonic probe having improved heat dissipation capability may be provided according to the present disclosure.

According to the present disclosure, a multi-row ultrasonic probe having improved safety may be provided by reducing the surface temperature thereof.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. An ultrasonic probe comprising:
a transducer configured to generate ultrasound and having multiple rows;
a kerf dividing the transducer into multiple rows; and
a heat dissipation member located inside the kerf and transferring heat generated by the transducer to the outside.

2. The ultrasonic probe of claim 1, wherein a width of the heat dissipation member is equal to or smaller than that of the kerf.

3. The ultrasonic probe of claim 1, wherein the heat dissipation member has a thermal conductivity of 0.5 W/m.K or greater.

4. The ultrasonic probe of claim 3, wherein the heat dissipation member comprises a coating layer covering an outer surface of the heat dissipation member and formed of a material comprising at least one of a non-metallic material, silicone, an epoxy resin, and parylene to reduce electrical conductivity of the heat dissipation member.

5. The ultrasonic probe of claim 1, further comprising a heat dissipation housing provided to cover an outer periphery of the transducer,
wherein the heat dissipation member is connected to the heat dissipation housing to transfer heat of the heat dissipation member to the heat dissipation housing.

6. The ultrasonic probe of claim 1, further comprising a matching layer provided on an upper surface of the transducer.

7. The ultrasonic probe of claim 6, wherein the kerf comprises:
a first kerf dividing the transducer into rows; and
a second kerf dividing the matching layer in the same direction as a direction in which the first kerf divides the transducer.

8. The ultrasonic probe of claim 7, wherein a height of the heat dissipation member is greater than a height of the transducer and equal to or smaller than a sum of heights of the transducer and the matching layer.

9. The ultrasonic probe of claim 7, further comprising a backing layer provided on a lower surface of the transducer.

10. The ultrasonic probe of claim 9, wherein the kerf further comprises a third kerf dividing the backing layer in the same direction as a direction in which the first kerf divides the transducer.

11. The ultrasonic probe of claim 10, wherein the height of the heat dissipation member is greater than the height of the transducer and equal to or smaller than a sum of heights of the transducer, the matching layer, and the backing layer.

12. The ultrasonic probe of claim 1, wherein the kerf is provided plural in number, and
the heat dissipation member is located inside each of the plurality of kerfs.

13. The ultrasonic probe of claim 12, wherein a plurality of heat dissipation members are located inside each of the plurality of kerfs.

14. The ultrasonic probe of claim 1, wherein a height of the heat dissipation member is greater than a width of the heat dissipation member.

15. The ultrasonic probe of claim 1, wherein the heat dissipation member comprises a plurality of heat dissipation wires arranged in parallel in directions direction crossing the elevation direction and the azimuth direction, respectively.
